# EUROPÄISCHE PATENTSCHRIFT

(11) **EP 1 589 113 B1**
(45) Veröffentlichungstag und Bekanntmachung des Hinweises auf die Patenterteilung: **21.09.2011**
(21) Anmeldenummer: 05002870.3
(22) Anmeldetag: 11.02.2005
(51) Int. Cl.: C12Q 1/18

(54) **Verfahren für die Empfindlichkeitsprüfung bakterieller Infektionserreger und Impfstämme des Geflügels**
Method for determining the sensitivity of bacterial pathogens and vaccine strains of poultry
Procédé pour déterminer la sensibilité de pathogènes bactériennes et de souches de vaccine dans la volaille

(30) Priorität: 19.04.2004 DE 202004006282 U
(43) Veröffentlichungstag der Anmeldung: 26.10.2005
(73) Patentinhaber: Lohmann Animal Health GmbH & Co. KG, 27472 Cuxhaven (DE)
(72) Erfinder: Rebeski, Dierk E., Dr. Dr., D-27476 Cuxhaven (DE); Schröder, Ilka, Dr., D-21765 Nordleda (DE); Iburg, Michael, Dr., D- 28359 Bremen (DE)
(74) Vertreter: Hauck Patent- und Rechtsanwälte

(56) Entgegenhaltungen:
- WO-A-94/16097
- PHILLIPS I ET AL: "DETERMINATION OF ANTIBIOTIC SENSITIVITIES BY THE SENSITITER SYSTEM" JOURNAL OF CLINICAL PATHOLOGY (LONDON), Bd. 31, Nr. 6, 1978, Seiten 531-535, XP009048879 ISSN: 0021-9746
- BLACKALL ET AL.: "Antimicrobial sensitivity testing of Australian isolates of Bordetella avium and the Bordetella avium-like organisms", AUSTRALIAN VETENINARY JOURNAL, vol. 72, 3 March 1995 (1995-03-03), pages 97-100,

## Beschreibung

Die Erfindung bezieht sich auf ein Verfahren zur Unterscheidung und Feststellung von Feldstämmen und Impfstämmen bakterieller Infektionserreger des Geflügels, insbesondere Huhn, Pute und Ente.

Für die in-vitro-Empfindlichkeitsprüfung bakterieller Infektionserreger stehen dem mikrobiologischen Untersuchungslabor zwei Methoden zur Verfügung: Das Diffusions- und das Dilutionsverfahren. Das am häufigsten angewendete Diffusionsverfahren ist der Plättchen-Agardiffusionstest. Die Resistenzbestimmung wird auf Festmedium durchgeführt und der Hemmhof-Durchmesser (HHD) für eine im Plättchen absorbierte konstante Wirkstoffkonzentration bewertet. Die HHD-Grenzwerte können den Kategorien "sensibel", "intermediär" und "resistent" zugeordnet werden.

Ebenfalls auf dem Diffusionsverfahren basierend kombiniert der E-Test die Verwendung von Festmedium mit einem Wirkstoffgradienten auf einem Papierstreifen. Die Ausbildung einer ellipsoiden Hemmzone erlaubt die Bestimmung der minimalen Hemmkonzentration (MHK).

Im Dilutionsverfahren wird im Reihenverdünnungstest das Wachstum der Testkeime in Flüssigmedium in Gegenwart unterschiedlicher Konzentrationen des Wirkstoffes getestet. Die Empfindlichkeit des Erregers gegenüber den verschiedenen Wirkstoffkonzentrationen wird durch die Bestimmung der MHK bewertet (primäres Verfahren). Für die humanmedizinische Diagnostik ist der Reihenverdünnungstest als standardisierte Untersuchungsmethode für einen hohen Probendurchsatz etabliert. In der diagnositischen Veterinärmedizin wird dieser bevorzugt für die aus Schwein und Rind isolierten bak teriellen Infektionserreger eingesetzt, um die aktuelle Resistenzsituation bakterieller Erreger im Tierbestand zu beurteilen. Zusätzlich unterstützt das quantitative Verfahren den Tierarzt in der Wahl des Wirkstoffes und somit den Therapieerfolg.

Standardisierbare und automatisierbare Verfahren für die Empfindlichkeitsprüfung bakterieller Infektionserreger des Geflügels stehen zwar zur Verfügung. Entsprechende Verfahren gibt es jedoch nicht für die Unterscheidung und Feststellung von Feldstämmen und Impfstämmen bei Geflügel. Dies auch nicht in Kombination mit der Erstellung eines Antibiogramms.

Phillips I. et al., J. Clin. Pathol., vo1.31, 1978, Seiten 531-535 offenbart ein Verfahren zum Bestimmen der minimalen Hemmkonzentration (MIC - minimum inhibitory concentration) mittels einer Mikrotiterplatte, die hierfür getrocknete Antibiotika in geeigneten Konzentrationen enthält, wobei Organismen in flüssigem Kulturmedium aufgegeben und über Nacht inkubiert werden. Das Verfahren ist für die Überprüfung von Organismen gedacht, die dem Blut des Patienten entnommen werden, d.h. für Humananwendungen bzw. klinischen Anwendungen (Seite 531, linke Spalte, Mitte und rechte Spalte, unten).

Das Verfahren ermöglicht keine Unterscheidung und Feststellung von Feldstämmen und Impfstämmen bei Geflügel.

Blackall et al., "Antimicrobial sensitivity testing of Australian isolates of Bordetella avium and the Bordetella avium-like organisms", Australian Veteninary Journal, Vol. 72, No. 3, March 1995, pages 97-100 offenbart ein Verfahren zur Empfindlichkeitsprüfung von Bordetella avium und verwandten Bakterien des Geflügels unter Verwendung einer Mikrotiterplatte mit Antibiotika, bei dem eine vom Geflügel isolierte Bakterienkultur in Suspension in einer Nährlösung mit verschiedenen Antibiotika inkubiert wird, und das Wachstum von Bakterien ermittelt wird.

Davon ausgehend liegt der Erfindung die Aufgabe zugrunde, ein Verfahren zur Bestimmung und Unterscheidung von Feldstämmen und Impfstämmen bakterieller Infektionserreger des Geflügels zu liefern.

Die Aufgabe wird durch ein Verfahren mit den Merkmalen des Anspruches 1 gelöst. Vorteilhafte Ausgestaltungen des Verfahrens sind in den Unteransprüchen angegeben.

Die erfindungsgemäße Verfahren zur Unterscheidung und Feststellung von Feldstämmen und Impfstämmen bakterieller Infektionserreger des Geflügels unter Verwendung einer Vorrichtung mit einer eine Vielzahl Vertiefungen aufweisenden Mikrotiterplatte und mindestens einer Vertiefung mit einer ein Antibiotikum eines Markers, d.h. Marker-Antibiotikum, eines Impfstammes für die Impfung von Geflügel umfassenden Beschichtung wird mindestens eine vom Geflügel isolierte Bakterienkultur in einer Nährlösung in eine Vertiefung mit einer ein Antibiotikum eines Markers eines Impfstammes für die Impfung von Geflügel umfassenden Beschichtung gegeben, inkubiert, das Wachstum der Bakteriumkultur in der Vertiefung überprüft und anhand des Wachstums im Vergleich zum Wachstum von Feldstämmen das Vorhandensein von Feldstämmen oder Impfstämmen in der Bakterienkultur ermittelt.

Die in mindestens einer Vertiefung der Mikrotiterplatte vorhandene Beschichtung ermöglicht eine Unterscheidung zwischen einem krankheitserregenden Feldstamm eines bakteriellen Infektionserregers und dem für die Impfung herangezogenen, gegenüber dem Feldstamm unterscheidbaren Impfstamm. Die Unterscheidung basiert darauf, daß der Impfstamm einen Marker aufweist, der mit einer erhöhten Resistenz oder einer erhöhten Empfindlichkeit des Impfstammes gegen ein Antibiotikum einhergeht. Die Beschichtung ist im festen Zustand und ist in einer dem Fachmann bekannten Weise aufgebracht. Wird nun in eine das Antibiotikum umfassende Beschichtung aufweisende Vertiefung der Mikrotiterplatte eine von Geflügel isolierte Bakterienreinkultur in Suspension in einer geeigneten Nährlösung aufgegeben, so werden die Antibiotika rehydriert und gewinnen ihre volle Wirksamkeit zurück. Nach einer Inkubation (z.B. bei 35 bis 37 °C über 18 bis 24 Stunden) ist das Wachstum der Bakterien meßbar. Anhand des Wachstums der Bakterien kann festgestellt werden, ob es sich hierbei um einen Infektionserreger oder um einen Impfstamm handelt. Das Wachstum resistenter Impfstämme wird nämlich im Vergleich zu Feldstämmen durch die Beschichtung nicht beeinträchtigt. Hingegen wird das Wachstum empfindlicher Impfstämme im Vergleich zu dem von Feldstämmen durch die Beschichtung beeinträchtigt. Ein Wachstum der Bakterien zeigt sich in einer zunehmenden Trübung der Suspension. Diese ist visuell oder mittels eines Fotometers objektiv meßbar ohne Interpretationsspielraum durch den Anwender. Dies ermöglicht die Kontrolle der Aufnahme von Impfstoffen durch das Geflügel und der Bekämpfung der Feldstämme durch einen standardisierten Test. Die Vorrichtung kann auch für automatische Bearbeitung verwendet werden.

Gemäß einer Ausgestaltung weist mindestens eine Vertiefung eine ein Antibiotikum eines Markers eines Salmonella-Impfstammes umfassende Beschichtung auf. Hierbei handelt es sich z.B. um Antibiotika, die Marker von Salmonella-Stoffwechseldrift-Mutanten bzw. -Impfstämmen sind. Des weiteren handelt es sich hierbei z.B. um bestimmte Antibiotika, denen gegenüber der Salmonella-Impfstamm aufgrund eines Envelope-Markers eine erhöhte Sensibilität aufweist. Einschlägige Salmonella-Impfstämme sind in dem europäischen Patent EP 0 263 528 B1 (insbesondere in den Ansprüchen und in der Tabelle in den Zeilen 26 bis 49 der Seite 5) und in dem europäischen Patent EP 0 642 796 B1 (insbesondere in den Ansprüchen und der Tabelle auf Seite 6, Zeilen 30 bis 46) angegeben. Die Offenbarung der beiden europäischen Patente betreffend Salmonella-Stoffwechseldrift-Mutanten und Salmonella-Envelope-Mutanten wird in die vorliegende Anmeldung einbezogen. Entsprechend gemarkerte Geflügel-Lebendimpfstoffe werden von der Anmelderin unter den Markenbezeichnungen Salmonella VAC®T und Salmonella VAC®E vermarktet.

Gemäß einer Ausgestaltung der Erfindung weist mindestens eine Vertiefung eine Beschichtung umfassend ein Antibiotikum ausgewählt aus den Antibiotika Streptomycin (STRE), Rifampicin (RAM), Erythromycin (ERYD) auf. Streptomycin und Rifampicin sind Stoffwechseldrift-Marker von Impfstämmen, die in der EP 0 263 528 B1 und EP 0 642 796 B1 angeführt sind. Die in der EP 0 642 796 B B1 angeführten Impfstämme weisen einen Envelope-Marker auf, der diesen eine erhöhte Sensibilität gegenüber Erythromycin verleiht. Salmonella VAC®T und VAC®E sind auf der Grundlage von Impfstämmen aus den beiden Patenten entwickelt worden.

Gemäß einer Ausgestaltung umfaßt mindestens eine Vertiefung eine ein therapeutisch relevantes Antibiotikum für Geflügel umfassende Beschichtung. Dies ermöglicht es, die Wirksamkeit eines für Geflügel therapeutisch relevanten Antibiotikums gegen eine von Geflügel isolierte Bakterienkultur zu überprüfen. Auch bei diesem Test wird eine isolierte Bakterienkultur in Suspension in einer geeigneten Nährlösung in die Vertiefung aufgegeben und inkubiert. Schließlich wird das Wachstum der Bakterienkultur anhand der Trübung überprüft. Hieraus sind Änderungen der Empfindlichkeit und des Widerstandes von Bakterienisolaten gegen Antibiotika ersichtlich. Diese Informationen können für die Feststellung des Gesundheitszustandes von Geflügel, für ein Resistenzmonitoring und für die therapeutische Behandlung durch den Veterinär genutzt werden.

Die Beschichtung umfaßt als therapeutisch relevantes Antibiotikum ein Antibiotikum, das für die Behandlung von Infektionserkrankungen von Geflügel von besonderer Bedeutung ist bzw. häufig eingesetzt wird. Vorzugsweise sind mehrere Vertiefungen mit verschiedenen Beschichtungen mit einer Vielfalt therapeutisch relevanter Antibiotika versehen. Die therapeutische Relevanz von Antibiotika kann sich im Laufe der Zeit verändern. Neue therapeutisch relevante Antibiotika können hinzukommen. Die Erfindung bezieht sämtliche Antibiotika ein, die derzeit und in Zukunft für die therapeutische Behandlung von Geflügel von Bedeutung sind.

Eine Ausgestaltung weist mindestens eine Vertiefung mit einer Beschichtung umfassend ein Antibiotikum ausgewählt aus den Antibiotika Ampicillin (AMP), Ceftiofur (CET), Colistin (CST), Enrofloxacin (ENRO), Erythromycin (ERY), Gentamycin (GEN), Lincomycin (LIN), Neomycin (NEO), Oxacillin (OXA), Penicillin G (PEN), Spectinomycin (SPEC), Streptomycin (STRE), Tetracycline (TET), Tiamulin (TIA), Trimethoprim-sulfamethoxazole (T/S) auf. Es handelt sich hierbei um Antibiotika, die nach Feststellungen der Anmelderin derzeit für Geflügel von hoher therapeutischer Relevanz sind. Ceftiofur ist zwar für die Behandlung von Geflügel nicht zugelassen. Die Feststellung des Einsatzes von Ceftiofur ist jedoch für die Diagnostik von Interesse. Eine Platte mit mehreren Vertiefungen mit unterschiedliche Antibiotika aufweisenden Beschichtungen ermöglicht die Feststellung eines Antibiogramms für eine von Geflügel isolierte Bakterienkultur.

Hat das Antibiotikum in der Beschichtung eine vorgegebene Konzentration, ist eine quantitative Aussage über die Wirksamkeit des Antibiotikums gegen den Infektionserreger oder den Impfstamm möglich. Die Durchführung des Tests folgt vorzugsweise im wesentlichen der Dokumentation der Standarddurchführungskriterien, die vom National Commety for Clinical Laboratory Standards (NCCLS), Wayne, Pensilvania, USA, herausgegeben worden sind. Diese sind zu finden in: National Committee for Clinical Laboratory Standard (2002). Performance Standards for Antimicrobial Disk and Dilution Susceptibility Tests for Bacteria Isolated from Animals; Approved Standard - Second Edition. Document M31-A2, Vol. 22, No. 6, NCCLS, Wayne, PA, USA.

Gemäß einer weiteren Ausgestaltung enthalten die Beschichtungen verschiedener Aufnahmen dieselben Antibiotika in verschiedener Konzentration. Dies ermöglicht auf die MHK bezogene quantitative Angaben zur Wirksamkeit eines Antibiotikums gegen eine von Geflügel isolierte Bakterienkultur. Die Wirksamkeit des Antibiotikums in-vitro gegen einen bestimmten Infektionserreger kann anhand von MHK-Grenzwerten ermittelt werden. Diese werden entsprechend den klinischen Kategorien als empfindlich, intermediär und resistent angegeben.

Hierzu enthalten gemäß einer Ausgestaltung Beschichtungen verschiedener Aufnahmen das Antibiotikum in einer dem unteren Grenzwert und dem oberen Grenzwert entsprechenden Konzentration. Der untere Grenzwert entspricht einem MHK-Wert, welcher vollständig ein hinreichendes und sichtbares Bakterienwachstum verhindert, d.h. das Bakterienisolat ist empfindlich für das Antibiotikum. Der obere Grenzwert entspricht einem MHK-Wert, der nur teilweise ein hinreichendes und sichtbares Bakterienwachstum verhindert und eine intermediäre Wirksamkeit des Antibiotikums anzeigt. Deshalb zeigt die Messung von MHK-Werten oberhalb des oberen Grenzwertes die Resistenz des Bakterienisolats gegen ein bestimmtes Antibiotikum an. Die unteren Grenzwerte und oberen Grenzwerte werden jeweils der aktuellen Literatur entnommen. Für das Ausführungsbeispiel der Erfindung sind die Quellen in dem der Durchführung des Tests gewidmeten Teil der Beschreibung angegeben. Die Vertiefungen werden mit dem Wirkstoff bzw. Antibiotikum so beschichtet, daß die gewünschten Wirkstoffkonzentrationen bei Eingabe einer definierten Probenmenge in der Probe erreicht werden.

Gemäß einer weiteren Ausgestaltung weisen mehrere Vertiefungen dieselbe Beschichtung auf. Dies ermöglicht Vergleichsmessungen, welche die Sicherheit der Empfindlichkeitsprüfung erhöhen.

Eine Ausgestaltung weist mindestens eine Vertiefung ohne Beschichtung auf. Dies ermöglicht eine Vergleichsmessung an einer Bakterienkultur, deren Wachstum nicht beeinträchtigt ist.

Eine Ausgestaltung weist mehrere Gruppen Vertiefungen auf, wobei die Vertiefungen in den Gruppen verschiedene Beschichtungen und die Vertiefungen verschiedener Gruppen dieselben Beschichtungen aufweisen. Hierdurch werden erweiterte Vergleichsmessungen ermöglicht.

Gemäß einer weiteren Ausgestaltung hat die Mikrotiterplatte 96 Vertiefungen. Dieses Format erleichtert insbesondere das Arbeiten mit Handpipetten. Die Erfindung bezieht jedoch Mikrotiterplatten mit einer anderen Anzahl Vertiefungen ein, z.B. 384 oder 1536 Vertiefungen, die insbesondere für die Automatenbearbeitung geeignet sind.

Gemäß einer Ausgestaltung ist die Mikrotiterplatte in einem luftdichten Beutel verpackt und hierdurch wird die Wirksamkeit der Beschichtungen über einen längeren Lagerzeitraum bei Raumtemperatur (15 - 25 °C) gewährleistet. Hierzu enthält gemäß einer weiteren Ausgestaltung der Beutel ein Trockenmittel.

Nachfolgend wird die Erfindung anhand der anliegenden Zeichnungen eines Ausführungsbeispieles und einer zugeordneten Tabelle näher erläutert. In den Zeichnungen zeigen:

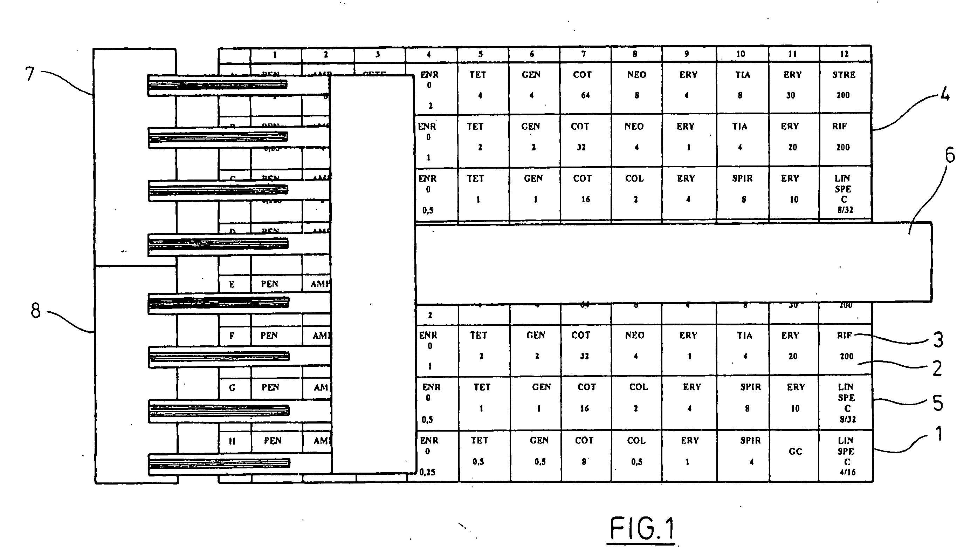

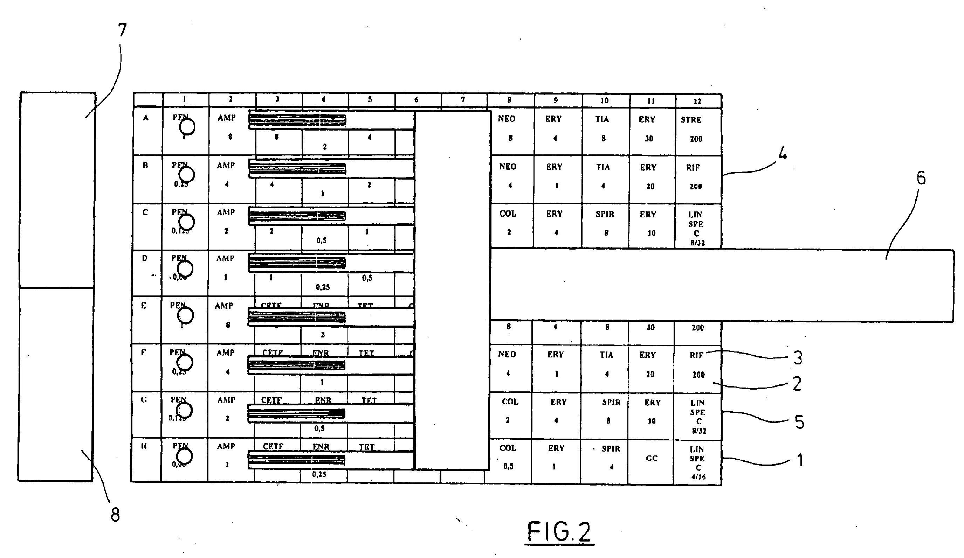

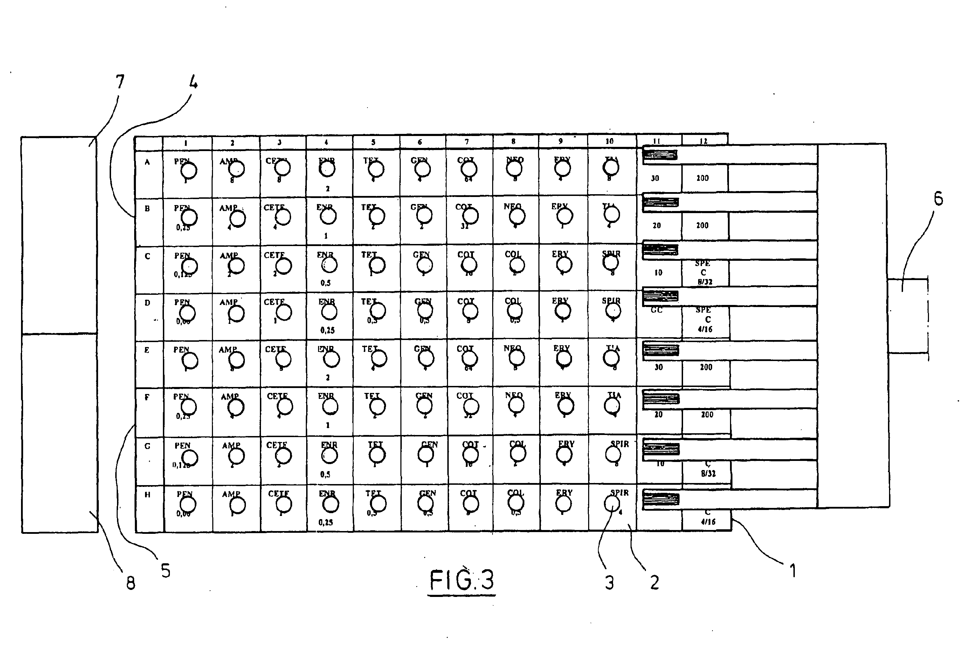

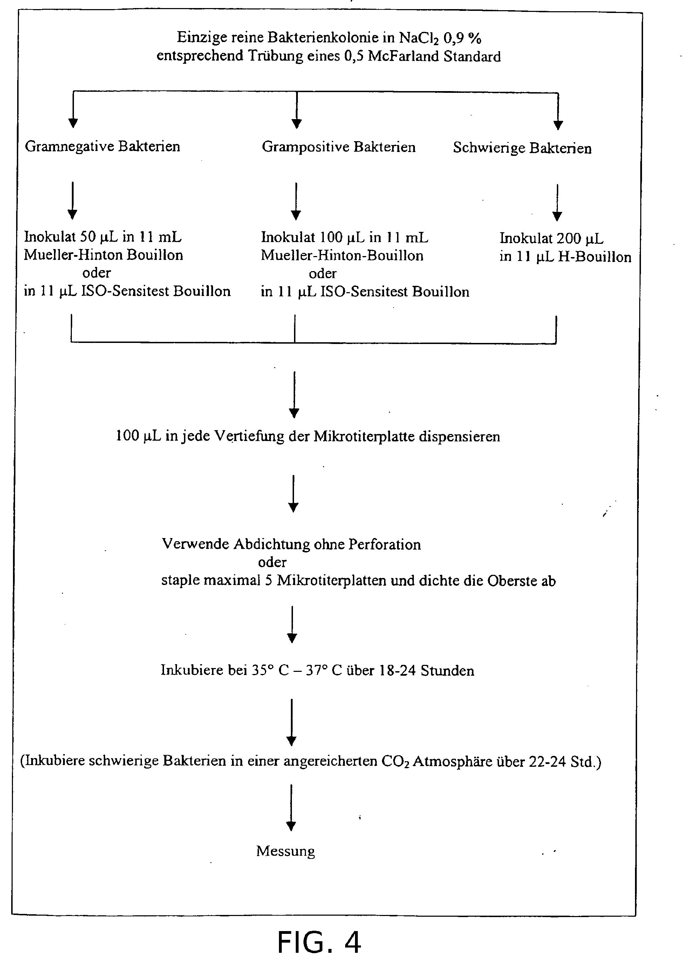

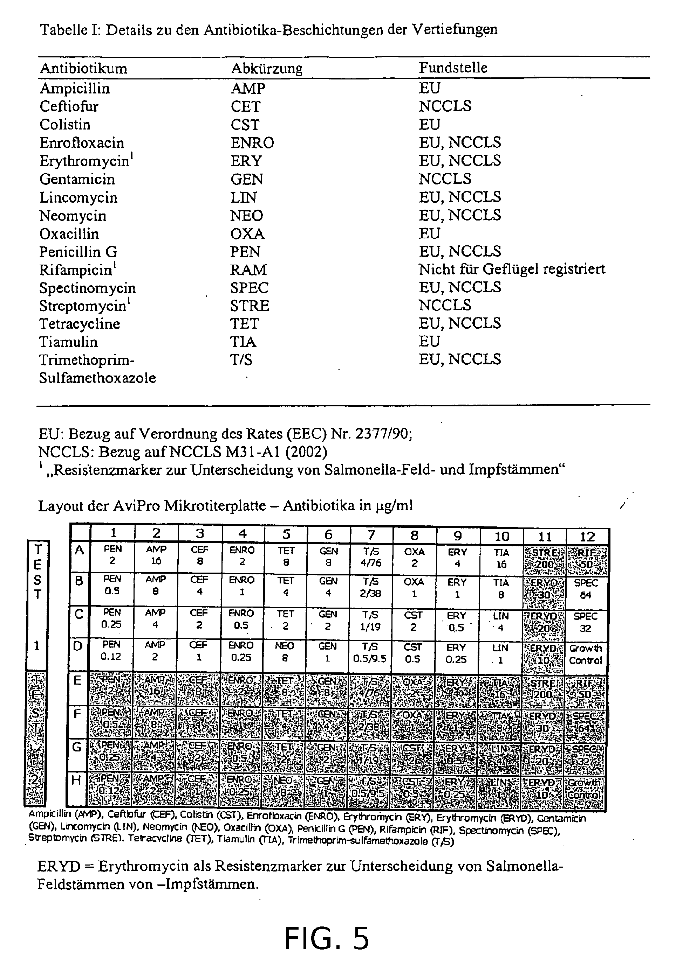

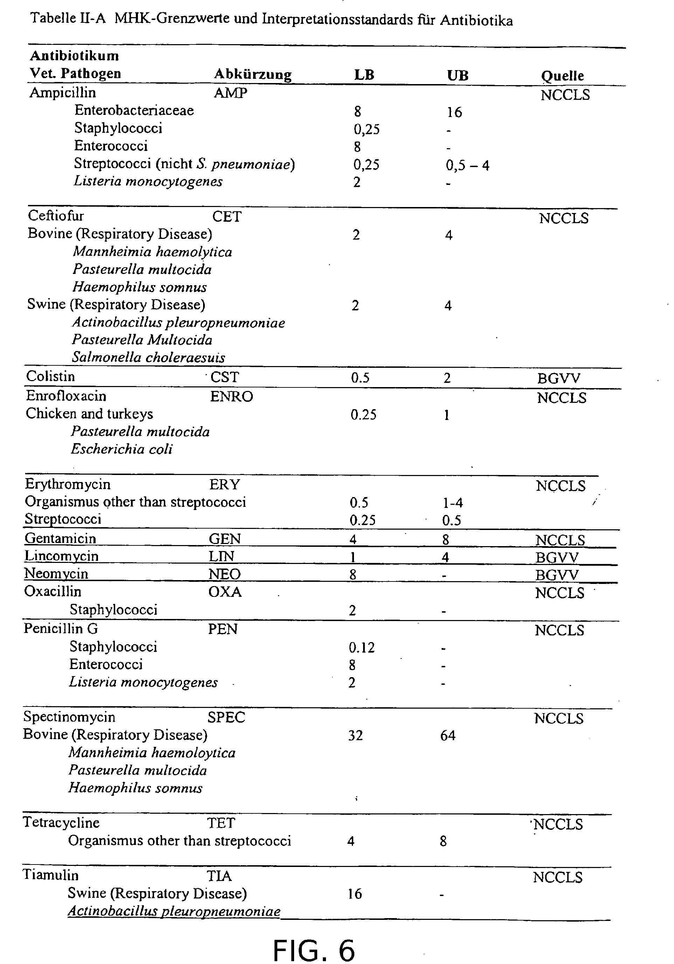

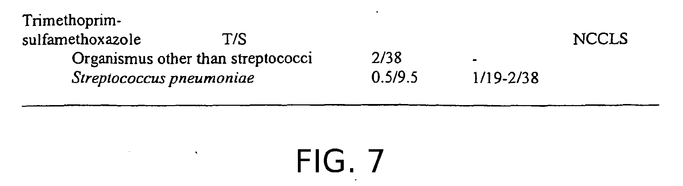

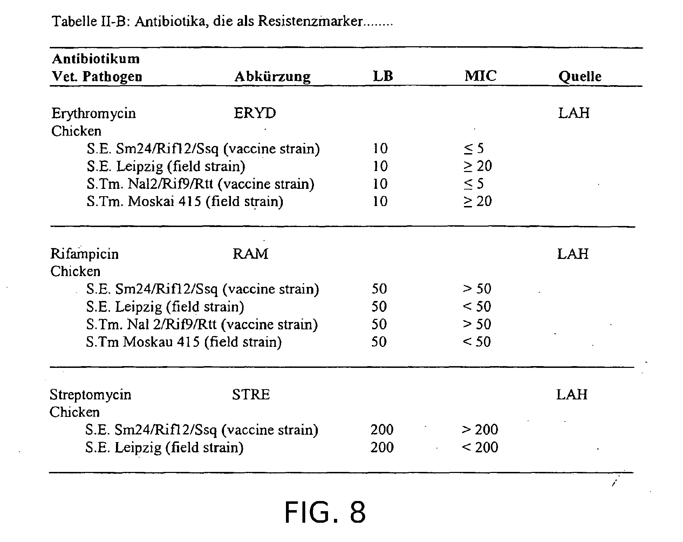

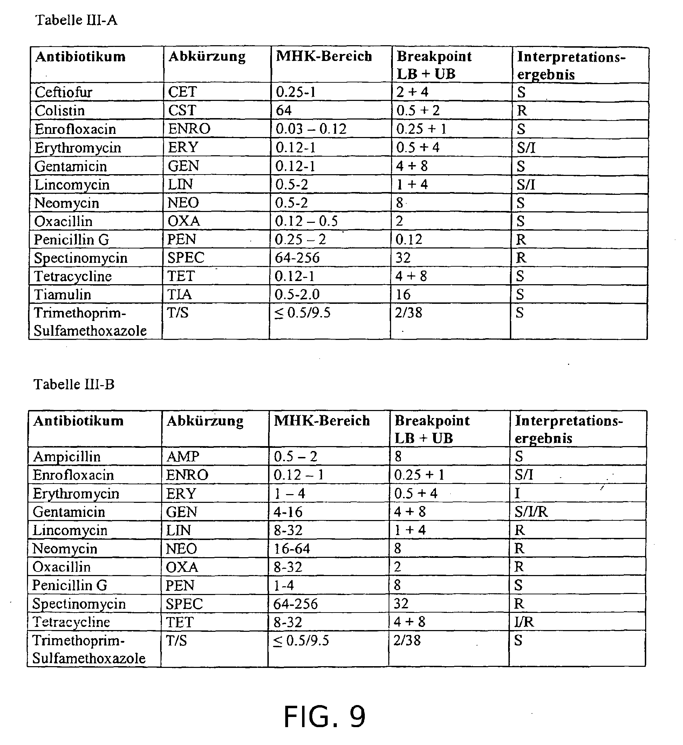

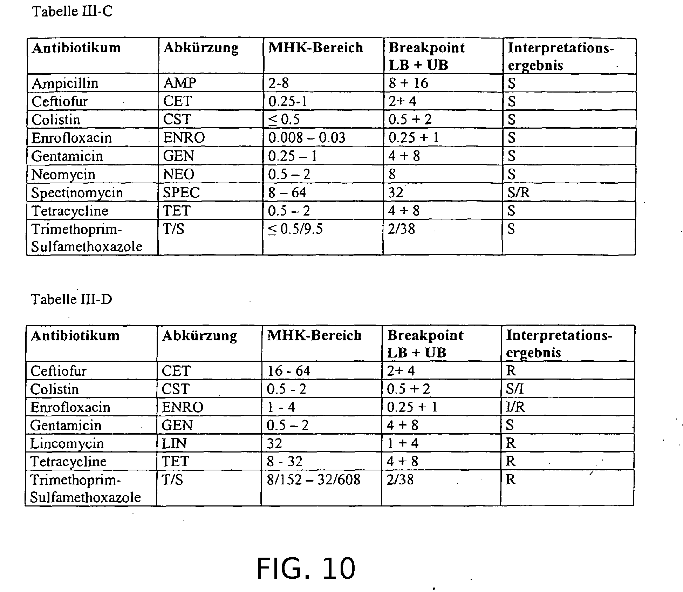

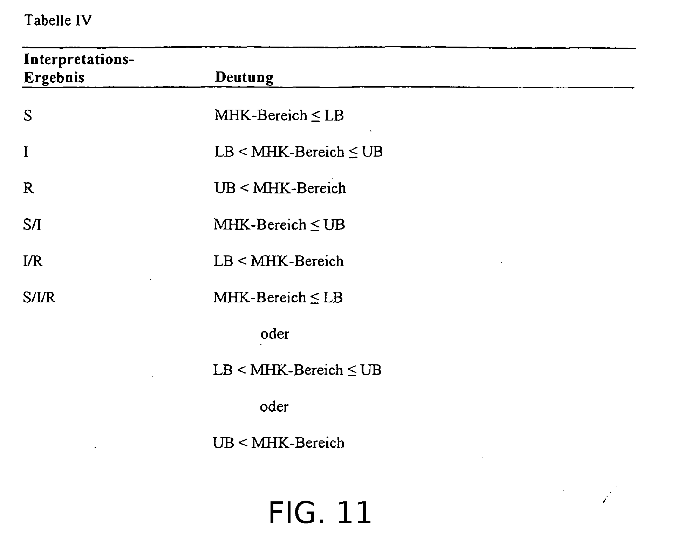

- Fig. 1 bis 3: eine Mikrotiterplatte beim Pipettieren einer Bakteriensuspension in mehreren Schritten in einer grobschematischen Draufsicht.

Gemäß Fig. 1 bis 3 hat eine Mikrotiterplatte 1 96 Vertiefungen 2. Die Vertiefungen 2 enthalten Beschichtungen 3, welche Antibiotika umfassen. Die Antibiotika sind in den Fig. 1 bis 3 mit Abkürzungen bezeichnet, die in der Tabelle 1 den Antibiotika zugeordnet sind.

In den Fig. 1 bis 3 und in der Tabelle I sind auch die Konzentrationen der Antibiotika in den Vertiefungen 2 in Mikrogramm pro Milliliter angegeben. Bei den Konzentrationen handelt es sich um die Massen der jeweiligen Antibiotika bezogen auf das Volumen der in die Vertiefungen 2 einzupipettierenden Probe (z.B. 100 µl). In der Tabelle 1 sind ferner die Referenzen für die gewählten Konzentrationen angegeben.

Die Mikrotiterplatte 1 weist zwei Gruppen 4, 5 Vertiefungen 2 mit identischen Beschichtungen 3 auf. Die Gruppe 4 ist in den Zeichnungen in der oberen Hälfte der Mikrotiterplatte 1 und die Gruppe 5 ist in der unteren Hälfte gezeigt.

Mit Hilfe einer Achtkanal-Pipette 6 werden aus einem Zweikanal-Reservoir 7, 8 Bakteriensuspensionen 1 und 2 in die verschiedenen Vertiefungen 2 pipettiert. In den Fig. 1 bis 3 sind verschiedene Pipettierschritte dargestellt.

Einzelheiten der Durchführung des Tests sind in der nachfolgenden Beschreibung angegeben:

### TESTPRINZIP

Die Empfindlichkeitsbestimmung mit der Mikrotiterplatte 1 beruht auf der Rehydratisierung von Antibiotika durch Zugabe einer standardisierten Bakterien-Suspension. Das Ergebnis wird nach 18 - 24 Stunden Inkubation bei 35 - 37°C photometrisch mit dem MICRONAUT Skan oder einem anderen geeigneten Fotometer gemessen und mit der MICRONAUT Software ausgewertet oder visuell abgelesen und interpretiert.

### MIKROTITERPLATTE

Jede einzelne Mikrotiterplatte 1 ist in einem Aluminiumbeutel eingepackt zusammen mit einem Trocknungsmittel. Die Testplatte umfaßt Antibiotika mit verschiedenen Konzentrationen, die bevorzugt bei Geflügel zum Einsatz kommen (siehe Anhang: Tabelle I und das AviPro® Plattenlayout). Die Mikrotiterplatte 1 ermöglicht zwei Empfindlichkeitstests pro Platte. Jeder Test erfolgt in bis zu 48 Vertiefungen 2, die eine Palette von 16 Antibiotika und eine Wachstumskontrolle umfassen. Die Vertiefungen 2 der Reihen A bis D sind für Test 1 bestimmt und die Vertiefungen der Reihe E bis H für Test 2. Ferner gibt es eine Klebefolie zum Abdichten der Platte.

### GEEIGNETE ZUSATZPRODUKTE UND LABORMATERIALIEN

- Mueller-Hinton Bouillon oder ISO-Sensitest Buillon modifiziert ohne Salz
- H-Bouillon
- NaCl 0,9 % pH 5,5 bis 6,5 bei 37 °C
- 1-Kanal-Reservoir
- 2-Kanal-Reservoir
- Mehrkanalpipette (8- oder 12-Kanal)
- Pipettenspitzen
- MICRONAUT Skan (# L5-120-001) oder anderes geeignetes Fotometer
- MICRONAUT Software (# U8-305-001)
- McFarland Standard 0,5
- Blutagarplatte
- Brutschrank 37⁰C
- Impfösen
- Markierstift
(Produkte mit Artikelnummer sind bei der MERLIN Diagnostika GmbH erhältlich. MICRONAUT ist eine Marke der Merlin Diagnostik GmbH.)

### MEDIEN UND REAGENZIEN

| | |
|---|---|
| Reagenz | Bestandteile |
| NaCl 0,9 % 11 | Natriumchlorid |
| Iso-Sensitest Buillon modifiziert (100 Röhrchen á 11 ml) | Iso-Sensitest Buillon Di-Natriumphosphat |
| H-Buillon (100 Röhrchen á 11 ml) | Haematin, NaOH, Tween 80, Pyridoxal, β-Nicotinamid, Adenindinukleotid, Columbia Buillonbasis, Glukose, Hefeextrakt, Neopepton, Agarose Typ IIA |
| Mueller-Hinton II | Rinderextrakt, saure Kasein-Hydrolysat |
| Bouillon | Stärke |

### TESTDURCHFÜHRUNG

### 1. GRAM PRÄPARAT:

- Grampositive Bakterien erscheinen blau, gramnegative Bakterien rot. Es wird empfohlen, die Gramfärbung nach entsprechendem Standardprotokoll oder den Empfehlungen auf den Herstellerkits zu ermitteln.

### 2. HERSTELLUNG DES INOKULUMS

- Ein Röhrchen mit 5 ml NaCl 0,9 % pH 5,5 bis 6,5 bereitstellen.
- Mehrere einzeln liegende Kolonien einer 18 - 24 Stunden alten Reinkultur vom Blutagar (ohne Zusatz) abnehmen.
- Die Kolonien in 5 ml NaCl 0,9 % gut homogenisieren, bis die Trübung einem McFarland von 0,5 entspricht.

### 3. INOKULATION IN BOUILLON

3.1 Mueller-Hinton II Bouillon oder modifizierte ISO-Sensitest-Bouillon
   Für gramnegative Bakterien werden 50 µl der Bakteriensuspension in 11 ml Mueller-Hinton II Bouillon oder modifizierter ISO-Sensitest Bouillon pipettiert und gut homogenisiert.
   Für grampositive Bakterien werden 100 µl der Bakteriensuspension in 11 ml Mueller-Hinton II Bouillon oder modifizierter ISO-Sensitest-Bouillon pipettiert und gut homogenisiert.
3.2 H-Bouillon
   Für schwierig zu kultivierende Bakterien werden 200 µl der Bakteriensuspension in 11 ml vorgewärmter H-Bouillon transferiert und homogenisiert.

### 4. BEIMPFUNG (Bouillon-Mikrodilution)

- Mikrotiterplatte max. 30 Minuten vor Beimpfung aus der Einzelverpackung entnehmen und das Trockenmittel verwerfen.
- Die Testplatte beschriften.
- Die vorbereitete Suspension gemäß der Plattenbelegung in ein 2-Kanal-Reservoir geben.
- Die Beimpfung der Mikrotiterplatte erfolgt manuell mit einer Mehrkanalpipette, 100 µl je Vertiefung.

### 5. VERSIEGELUNG UND INKUBATION

5.1 Mueller-Hinton II Bouillon oder modifizierte ISO-Sensitest-Bouillon
   - Nach dem Beimpfen die Testplatten mit einer unperforierten Abklebefolie verschließen oder mit einer unbenutzten Mikrotiterplatte abdecken (max. 5 Testplatten stapeln).
   - Testplatte 18―24 Stunden bei 35 -37°C inkubieren.
5.2 H-Bouillon
   - Nach dem Beimpfen der Testplatten mit einer unbenutzten Mikrotiterplatte abdecken oder mit einer perforierten Abdeckfolie verschließen.
   - Testplatte 22 bis 24 Stunden bei 35 bis 37 °C im Inkubator mit CO₂-Begasun bebrüten.

### 6. ABLESUNG

- Abklebefolie entfernen.
- Testplatte von unten abwischen.
- Ablesung der Testplatte mit dem Fotometer oder visuell.

### 7. AUSWERTUNG

Das Bakterienwachstum wird anhand einer photometrischen Messung bei einer Wellenlänge von 620 nm oder 690 nm - je nach verwendeter Bouillon - bestimmt.

Das Ergebnis wird mit Hilfe der MICRONAUT Software ausgewertet, interpretiert und auf seine Plausibilität überprüft. Die Wachstumskontrolle muss bewachsen (trüb) sein, anderenfalls muss der Test wiederholt werden. Bei der visuellen Ablesung sollten die Ergebnisse auf einem Plattenbelegungsplan protokolliert werden. Das Testergebnis kann am Bildschirm oder auf dem Befundausdruck angeschaut werden.

### 8. INTERPRETATION DER ERGEBNISSE

Breakpoint Interpretation
Für die Antibiotika, die in vier oder weniger aufeinanderfolgenden Konzentrationen oder in nicht aufeinanderfolgenden Konzentrationen getestet werden, werden interpretierende Kategorien, nämlich empfänglich, intermediär und resistent bestimmt, auf der Basis von niedrigen und oberen Breakpoint MHK-Werten (Siehe im Anhang: Tabelle IIA und IIB, Tabellen IIIA, IIIB, IIIC und IIID und Tabelle IV). Der niedrige Breakpoint (LB) oder untere Grenzwert ist representiert bei dem MHK-Wert, welcher ein hinreichendes und sichtbares bakterielles Wachstum verhindert, d.h. das Bakterienisolat ist empfänglich für das Antibiotikum. Dieser obere Breakpoint (UB) oder obere Grenzwert ist representiert durch den MHK-Wert, welcher teilweise hinreichendes und sichtbares bakterielles Wachstum verhindert und damit eine intermediäre Aktivität gegenüber dem Antibiotikum aufzeigt. Deshalb zeigt ein MHK-Wert oberhalb des oberen Grenzwerts (UB) eine Resistenz der Bakterienkolonie gegenüber dem Antibiotikum. Mit Hilfe der MICRONAUT-Software können MHK-Werte automatisch analysiert und interpretiert werden für ein gegebenes Bakterienisolat und Antibiotikum. Die Meßergebnisse und die entsprechende klinische Interpretation einschließlich des MHK-Bereichs können dem Tierarzt für die Therapie empfohlen werden.

Breakpoint-Interpretation von Antibiotika:

| | Visuelle | Ablesung | Automatisierte | Ablesung (OD) | Klinische Interpretation |
|---|---|---|---|---|---|
| | LB | UB | LB | UB | |
| Bakterienwachstum | - | - | < 0,1 | < 0,1 | Sensibel (S) |
| | + | - | ≥0,1 | < 0,1 | Intermediär (I) |
| | + | + | ≥ 0,1 | ≥0,1 | Resistent (R) |

| | | | | | |
|---|---|---|---|---|---|
| OD = optischer Dichtewert analysiert mit der MICRONAUT-Software; LB = niedrige Breakpoint-Konzentration; UB = obere Breakpoint-Konzentration; + Symbol = Vertiefung, die die Gegenwart von Bakterien anzeigt; - Symbol = Vertiefung, die die Abwesenheit von Bakterien anzeigt. ¹ Intermediäre Kategorie ist noch nicht für sämtliche Antibiotika bestimmt worden. ¹ niedrige Breakpoint Konzentration ² hohe Breakpoint Konzentration | | | | | |

### 9. QUALITÄTSKONTROLLE

Die bakteriologische Qualitätskontrolle kann mit folgenden Stämmen durchgeführt werden.

| Stämme | ATCC Nr. | DSMZ Nr. |
|---|---|---|
| *Staphylococcus aureus subsp. aureus* | ATCC 29213 | DSM 2569 |
| *Escherichia coli* | ATCC 25922 | DSM 1103 |
| *Pseudomonas aeruginosa* | ATCC 27853 | DSM 1117 |
| *Enterococcus faecalis* | ATCC 29212 | DSM 2570 |

ATCC = American Type Culture Collection
DSMZ= Deutsche Sammlung von Mikroorganismen und Zellkulturen GmbH

### LITERATUR

- BGVV Tabelle Stand XII/99. Bewertung der Hemmhofdurchmesser und Grenzwertkonzentrationen der in der Veterinärmedizin zugelassenen Antibiotika.
- BioSafety in Microbiological and Biomedical Laboratories, HHS Publication No. (CDC) 93-8395, 3rd Edition, May 1993.
- National Committee for Clinical Laboratory Standards (2002). Performance standards for antimicrobial disk and dilution susceptibility tests for bacteria isolated from animals; approved standard - second edition. Document M31-A2, Vol. 22, No. 6, NCCLS, Wayne, PA, USA.
- Verordnung (EWG) Nr. 2377/90 des Rates vom 26. Juni 1990 zur Schaffung eines Gemeinschaftsverfahrens für die Festsetzung von Höchstmengen für Tierarzneimittelrückstände in Nahrungsmitteln tierischen Ursprungs. Letzte Aktualisierung: Verordnung (EG) Nr. 544/2003 der Kommission vom 27. März 2003.
Anhang:
- Flußbild der Testprozedur
- Tabelle I Details zu den Antibiotika-Beschichtungen der Aufnahmen derselben Mikrotiterplatte.
- Layout der AviPro Mikrotiterplatte
- Tabelle II-A: MHK-Grenzwerte und Interpretationsstandards für Antibiotika;
- Tabelle II-B: Antibiotika, die als Resistenzmarker verwendet werden, um Feldstämme von Salmonella Enteritidis (S.E.) und Salmonella Typhimurium (S.Tm.) von Stoffwechseldriftmutanten in Lebendimpfstoffen von Salmonella Vac E und Salmonella Vac T zu unterscheiden;
- Tabelle III-A: Bereich der MHK und Interpretationsergebnis von Antibiotika, die auf grampositive Referenzstämme Staphylococcus aureus ATCC 29213 getestet worden sind;
- Tabelle III-B: Bereich von MHK und Interpretationsergebnis für Antibiotika, die auf einen grampositiven Referenzstamm Enterococcus fecalis ATCC 29212 getestet worden sind.
- Tabelle III-C: Bereich von MHK und Interpretationsergebnis für Antibiotika, die auf einem gramnegativen Referenzstamm Escherichia coli ATCC 25922 getestet worden sind;
- Tabelle III-D: Bereich von MHK und Interpretationsergebnis für Antibiotika, die auf einem gramnegativen Teststamm Pseudumonas aeruginosa ATCC 27853 getestet worden sind;
- Tabelle IV: Deutung von Interpretationsergebnissen, die in den Tabelle III-A, III-B, III-C und III-D angegeben worden sind.

## Patentansprüche

1. Verfahren zur Unterscheidung und Feststellung von Feldstämmen und Impfstämmen bakterieller Infektionserreger des Geflügels unter Verwendung einer Vorrichtung mit einer eine Vielzahl Vertiefungen aufweisenden Mikrotiterplatte und mindestens einer Vertiefung mit einer ein Marker-Antibiotikum eines Impfstammes für die Impfung von Geflügel umfassenden Beschichtung, bei dem mindestens eine vom Geflügel isolierte Bakterienkultur in Suspension in einer Nährlösung in eine Vertiefung mit einer ein Marker-Antibiotikum eines Impfstammes für die Impfung von Geflügel umfassenden Beschichtung gegeben, inkubiert, das Wachstum der Bakterienkultur in der Vertiefung überprüft und anhand des Wachstums im Vergleich zum Wachstum von Feldstämmen das Vorhandensein von Feldstämmen oder Impfstämmen in der Bakterienkultur ermittelt wird.

2. Verfahren nach Anspruch 1, bei dem die Mikrotiterplatte mindestens eine Vertiefung mit einer ein Marker-Antibiotikum eines Salmonella-Impfstammes umfassenden Beschichtung aufweist.

3. Verfahren nach Anspruch 1 oder 2, bei dem die Mikrotiterplatte mindestens eine Vertiefung mit einer Beschichtung umfassend ein Antibiotikum ausgewählt aus den Antibiotika Streptomycin, Rifampicin und Erythromycin aufweist.

4. Verfahren nach einem der Ansprüche 1 bis 3, bei dem die Mikrotiterplatte mindestens eine Vertiefung mit einer ein therapeutisch relevantes Antibiotikum für Geflügel umfassenden Beschichtung aufweist, mindestens eine vom Geflügel isolierte Bakterienkultur in Suspension in einer Nährlösung in eine Vertiefung mit einer ein therapeutisch relevantes Antibiotikum für Geflügel umfassenden Beschichtung gegeben, inkubiert, das Wachstum der Bakterienkultur in der Vertiefung überprüft und Änderungen der Empfindlichkeit oder Resistenz der Bakterien gegen das therapeutisch relevante Antibiotikum ermittelt werden.

5. Verfahren nach Anspruch 4, bei dem die Mikrotiterplatte mindestens eine Vertiefung mit einer Beschichtung umfassend ein Antibiotikum ausgewählt aus den Antibiotika Ampicillin, Ceftiofur, Colistin, Enrofloxacin, Erythromycin, Gentamicin, Lincomycin, Neomycin, Oxacillin, Penicillin G, Spectinomycin, Streptomycin, Tetracycline, Tiamulin und Trimethoprim-sulfamethoxazole aufweist.

6. Verfahren nach einem der Ansprüche 1 bis 5, bei dem die Beschichtungen verschiedener Vertiefungen der Mikrotiterplatte dieselben Antibiotika in verschiedener Konzentration enthalten.

7. Verfahren nach Anspruch 6, bei dem die Beschichtungen verschiedener Aufnahmen der Mikrotiterplatte das Antibiotikum in einer dem unteren Grenzwert und dem oberen Grenzwert der minimalen Hemmkonzentration enthaltenden Konzentration enthalten.

8. Verfahren nach einem der Ansprüche 1 bis 7, bei der Beschichtungen verschiedener Vertiefungen der Mikrotiterplatte verschiedene Antibiotika enthalten.

9. Verfahren nach einem der Ansprüche 1 bis 8, bei der mehrere Vertiefungen der Mikrotiterplatte dieselbe Beschichtung aufweisen.

10. Verfahren nach einem der Ansprüche 1 bis 9, bei dem die Mikrotiterplatte mindestens eine Vertiefung ohne Beschichtung aufweist.

11. Verfahren nach einem der Ansprüche 1 bis 10, bei dem die Mikrotiterplatte mehrere Gruppen Vertiefungen aufweist, wobei die Vertiefungen in den Gruppen verschiedene Beschichtungen und die Vertiefungen verschiedener Gruppen dieselben Beschichtungen aufweisen.

12. Verfahren nach einem der Ansprüche 1 bis 11, bei dem die Mikrotiterplatte 96 Vertiefungen aufweist.

13. Verfahren nach einem der Ansprüche 1 bis 12, bei dem die Mikrotiterplatte in einem luftdichten Beutel verpackt ist.

14. Verfahren nach Anspruch 13, bei dem der Beutel ein Trockenmittel enthält.

15. Verfahren nach einem der Ansprüche 1 bis 14, bei dem das Wachstum der Bakterienkultur anhand der Trübung der Suspension ermittelt wird.

## Claims

1. Method for determining the sensitivity of field strains and vaccine strains of bacterial pathogens of poultry using a device with a microtiter plate having a plurality of wells and at least one well with a coating comprising a marker antibiotic of a vaccine strain for vaccinating poultry, wherein at least one bacteria culture isolated from poultry is added in suspension in a nutrient solution into a well with a coating comprising a marker antibiotic of a vaccine strain for vaccinating poultry, is incubated, the growth of the bacteria culture in the well is examined and the presence of field strains or vaccine strains in the bacteria culture is determined by comparison of the growth with the growth of field strains.

2. Method according to claim 1, wherein the microtiter plate has at least one well with a coating comprising a marker antibiotic of a salmonella vaccine strain.

3. Method according to claim 1 or 2, wherein the microtiter plate has at least one well with a coating comprising an antibiotic selected from the antibiotics streptomycin, rifampicin and erythromycin.

4. Method according to any one of claims 1 to 3, wherein the microtiter plate has at least one well with a coating comprising an antibiotic that is therapeutically relevant for poultry, at least one bacteria culture isolated from poultry is added in suspension in a nutrient solution into a well with a coating comprising an antibiotic that is therapeutically relevant for poultry, is incubated, the growth of the bacteria culture in the well is examined and changes of the sensitivity or resistance of the bacteria against the therapeutically relevant antibiotic are determined.

5. Method according to claim 4, wherein the microtiter plate has at least one well with a coating comprising an antibiotic selected from the antibiotics ampicillin, ceftiofur, colistin enrofloxacin, erythromycin, gentamicin, lincomycin, neomycin, oxacillin, penicillin G, spectinomecin, streptomycin, tetracycline, tiamulin and trimethoprim-sulfamethoxazole.

6. Method according to any one of claims 1 to 5, wherein the coatings of different wells of the microtiter plate contain the same antibiotics in different concentrations.

7. Method according to claim 6, wherein the coatings of different accommodations of the microtiter plate contain the antibiotic in a concentration corresponding to the lower limit value and the upper limit value of the minimal inhibitory concentration.

8. Method according to any one of claims 1 to 7, wherein the coatings of different wells of the microtiter plate contain different antibiotics.

9. Method according to any one of claims 1 to 8, wherein plural wells of the microtiter plate have the same coating.

10. Method according to any one of claims 1 to 9, wherein the microtiter plate has at least one well without coating.

11. Method according to any one of claims 1 to 10, wherein the microtiter plate has plural groups of wells, wherein the wells in the groups have different coatings and the wells of different groups have the same coatings.

12. Method according to any one of claims 1 to 11, wherein the microtiter plate has 96 wells.

13. Method according to any one of claims 1 to 13, wherein the microtiter plate is packed in an air-tight bag.

14. Method according to claim 13, wherein the bag contains a drying means.

15. Method according to any one of claims 1 to 14, wherein the growth of the bacteria culture is determined with the help of the turbidity of the suspension.

## Revendications

1. Procédé pour déterminer la sensibilité de souches de terrain et de souches de vaccine de pathogènes bactériennes dans la volaille en utilisant un dispositif avec une plaque de micro-titrage avec une multitude de puits et au moins un puits avec un revêtement comprenant un antibiotique marqueur d'une souche de vaccine pour vacciner de la volaille, dans lequel au moins une culture de bactéries isolée de volaille est additionnée en suspension dans une bouillon de culture dans un puits avec un revêtement comprenant un antibiotique marqueur d'une souche de vaccine pour vacciner de la volaille est incubée, et la croissance de la culture de bactéries dans le puits est examinée et la présence de souches de terrain ou de souches de vaccines dans la culture de bactéries est déterminée à l'aide de la croissance comparée à la croissance de souches de terrain.

2. Procédé selon la revendication 1, dans lequel la plaque de micro-titrage a au moins un puits avec un revêtement comprenant un antibiotique marqueur d' une souche de vaccine de salmonelles.

3. Procédé selon la revendication 1 ou 2, dans lequel la plaque de micro-titrage a au moins un puits avec un revêtement comprenant un antibiotique sélectionné des antibiotiques streptomycine, rifampicine et érythromycine.

4. Procédé selon l'une quelconque des revendications à 3, dans lequel la plaque de micro-titrage a au moins un puits avec un revêtement comprenant un antibiotique important pour la thérapie de volaille, au moins une culture de bactéries isolée de volaille est additionnée en suspension dans une bouillon de culture dans un puits avec un revêtement comprenant un antibiotique important pour la thérapie de volaille, est incubée, la croissance de la culture de bactéries dans le puits est examinée et des changements de la sensibilité ou de la résistance des bactéries contre l'antibiotique important pour la thérapie sont déterminés.

5. Procédé selon la revendication 4, dans lequel la plaque de micro-titrage a au moins un puits avec un revêtement comprenant un antibiotique sélectionné des antibiotiques ampicilline, ceftiofure, colistine, enrofloxacine, erythromycine, gentamicine, lincomycine, neomycine, oxacilline, penicilline G, spectinomecine, streptomycine, tetracycline, tiamuline et trimethoprime-sulfamethoxazole.

6. Procédé selon l'une quelconque des revendications à 5, dans lequel les revêtements de différents puits de la plaque de micro-titrage contiennent les mêmes antibiotiques en des concentrations différentes.

7. Procédé selon la revendication 6, dans lequel les revêtements d'accommodations différentes de la plaque de micro-titrage contiennent l'antibiotique en une concentration correspondante à la valeur limite inférieure et la valeur limite supérieure de la concentration inhibitrice minimale.

8. Procédé selon l'une quelconque des revendications à 7, dans lequel les revêtements de puits différentes de la plaque de micro-titrage contiennent des antibiotiques différents.

9. Procédé selon l'une quelconque des revendications à 8, dans lequel plusieurs puits de la plaque de micro-titrage ont le même revêtement.

10. Procédé selon l'une quelconque des revendications à 9, dans lequel la plaque de micro-titrage a au moins un puits sans revêtement.

11. Procédé selon l'une quelconque des revendications à 10, dans lequel la plaque de micro-titrage a plusieurs groupes de puits, chacun des puits dans les groupes ayant des revêtements différents et les puits de groupes différents ayant les mêmes revêtements.

12. Procédé selon l'une quelconque des revendications à 11, dans lequel la plaque de micro-titraee a 96 puits.

13. Procédé selon l'une quelconque des revendications à 13, dans lequel la plaque de micro-titrage est emballée dans un sac hermétique.

14. Procédé selon la revendication 13, dans lequel le sac contient un moyen de séchage.

15. Procédé selon l'une quelconque des revendications à 14, dans lequel la croissance de la culture de bactéries est déterminée à l'aide de la turbidité de la suspension.
